(19) Europäisches Patentamt
European Patent Office
Office européen des brevets

(11) EP 4 574 826 A1

(12) **EUROPEAN PATENT APPLICATION**
published in accordance with Art. 153(4) EPC

(43) Date of publication:
**25.06.2025 Bulletin 2025/26**

(21) Application number: **23879138.8**

(22) Date of filing: **18.10.2023**

(51) International Patent Classification (IPC):
***C07D 487/04*** *(2006.01)* ***A61K 31/4985*** *(2006.01)*
***A61P 25/00*** *(2006.01)* ***A61P 29/00*** *(2006.01)*

(52) Cooperative Patent Classification (CPC):
**A61K 31/4985; A61P 25/00; A61P 29/00; C07D 487/04**

(86) International application number:
**PCT/CN2023/125214**

(87) International publication number:
**WO 2024/083150 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.10.2022 CN 202211282020**
**11.10.2023 CN 202311315851**

(71) Applicant: **Changchun Genescience Pharmaceutical Co., Ltd.**
**Changchun, Jilin 130012 (CN)**

(72) Inventors:
- **SHEN, Guangyuan**
  **Changchun, Jilin 130012 (CN)**
- **HUANG, Yue**
  **Changchun, Jilin 130012 (CN)**
- **JIN, Lei**
  **Changchun, Jilin 130012 (CN)**
- **WANG, Siqin**
  **Changchun, Jilin 130012 (CN)**
- **WANG, Guocheng**
  **Changchun, Jilin 130012 (CN)**

(74) Representative: **Gille Hrabal Partnerschaftsgesellschaft mbB**
**Patentanwälte**
**Brucknerstraße 20**
**40593 Düsseldorf (DE)**

(54) **CRYSTAL FORM OF NK3R ANTAGONIST, AND PREPARATION METHOD THEREFOR AND USE THEREOF**

(57) Disclosed in the present invention are a crystal form A of the NK3R antagonist represented by formula (I), and a preparation method therefor and the use thereof. The X-ray powder diffraction pattern of the crystal form A comprises characteristic peaks at 10.40±0.20°, 11.79 ±0.20°, 19.51±0.20° and 20.81±0.20° 2θ, as determined by using Cu-Kα radiation. The crystal form A provided in the present invention has stable properties, weak hygroscopicity, is suitable for long-term storage, is suitable for drug formation, and can effectively inhibit NK3R.

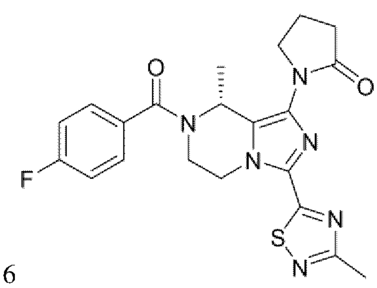

Chem 6 (I)

EP 4 574 826 A1

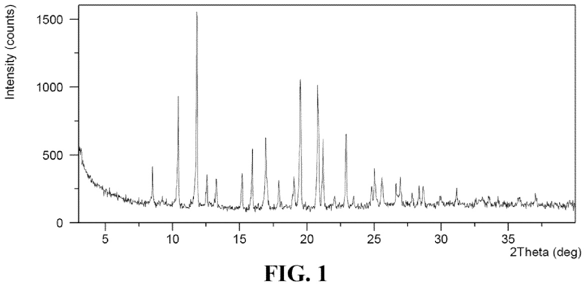


FIG. 1

## Description

The present application claims priority to two earlier applications as follows: Patent Application No. 202211282020.3 titled "CRYSTAL FORM OF NK3R ANTAGONIST, PREPARATION METHOD THEREFOR, AND USE THEREOF" and filed with the China National Intellectual Property Administration on 19 October 2022 and Patent Application No. 202311315851.0 titled "CRYSTAL FORM OF NK3R ANTAGONIST, PREPARATION METHOD THEREFOR, AND USE THEREOF" and filed with the China National Intellectual Property Administration on 11 October 2023. The full texts of the earlier applications are incorporated herein by reference.

## Technical Field

The present invention belongs to the field of pharmaceutical compounds, and specifically relates to a crystal form of a NK3R antagonist, a preparation method therefor, and use thereof.

## Background

Tachykinin receptors are targets of a family of structurally related peptides collectively named "tachykinins" that include substance P (SP), neurokinin A (NKA), and neurokinin B (NKB). The tachykinins are synthesized in central nervous system (CNS) and peripheral tissues, where they exert a variety of bioactivities. At present, there are three known tachykinin receptors named neurokinin-1 (NK-1) receptor, neurokinin-2 (NK-2) receptor, and neurokinin-3 (NK-3) receptor. The tachykinin receptors belong to rhodopsin-like G protein-coupled receptors. The SP has the highest affinity and is considered as an endogenous ligand for the NK-1 receptor, the NKA is an endogenous ligand for the NK-2 receptor, and the NKB is an endogenous ligand for the NK-3 receptor. The NK-1 receptor, the NK-2 receptor, and the NK-3 receptor have been identified in different species. The NK-1 receptor and the NK-2 receptor are expressed in various peripheral tissues, the NK-1 receptor is also expressed in the CNS, and the NK-3 receptor is mainly expressed in the CNS.

Neurokinin receptors mediate a variety of biological effects stimulated by the tachykinins, including transmitting excitatory neuron signals (e.g., pain) in the CNS and the periphery, modulating smooth muscle contractile activity, modulating immune responses and inflammatory responses, and inducing hypotensive effects and stimulating secretion from endocrine glands and exocrine glands by dilating peripheral vasculature.

The NK-3 receptor is encoded by TACR3 gene, and is involved in the regulation of hypothalamus -pituitary-gonad axis. TACR3 gene knockout or mutant mice all showed abnormal development of reproductive organs, low level of sex hormones, and severe reduction of reproductive capacity. Carrying a mutation in the TACR3 gene can cause abnormal gonadotropin release in patients, resulting in sexual immaturity and infertility in patients, and a considerable part of familial hypogonadism is caused by TACR3 gene mutations.

Kisspeptin/neurokinin B/dynorphin (KNDy) neurons are involved in gonadotropin-releasing hormone (GnRH) signaling pathway, and promotes estrogen production through GnRH neuron-pituitary-sex organ pathway, and this signaling pathway is regulated by a negative feedback mechanism, so as to keep hormone levels in vivo within a certain reasonable range. At the same time, KNDy neurons are also associated with thermoregulatory signaling pathway, bind to the NK-3 receptor on the median preoptic nucleus by releasing NKB ligands, and promote sweating and vasodilation by inhibiting shivering and vasoconstriction to regulate body temperature within a certain range. In menopausal women, due to the decrease of in vivo estrogen levels, the negative feedback mechanism is lost, resulting in overactivation of KNDy neurons and the release of a large amount of endogenous NKB ligands, which bind to the NK-3 receptors on the median preoptic nucleus, and leading to symptoms, such as sweating, vasodilation, and hot flash. Therefore, the development of an antagonist targeting the NK-3 receptor on the KNDy neurons and median preoptic nucleus is expected to have positive therapeutic effects on the symptoms of hot flash.

In the CNS, the NK-3 receptor is expressed in regions including medial prefrontal cortex, hippocampus, thalamus, and amygdala. In addition, the NK-3 receptor is expressed on dopaminergic neurons. Activation of the NK-3 receptor has been proved to modulate the release of dopamine, acetylcholine, and serotonin, suggesting the therapeutic utility of NK-3 receptor modulators in the treatment of various conditions, including psychotic disorder, anxiety disorder, depression, schizophrenia, obesity, pain, or inflammation.

Those skilled in the art have always been committed to solving a technical problem of developing a NK-3R antagonist with a novel structure and a pharmaceutical solid form suitable for preparing a drug from such a compound, such as a solid form with improved stability, hygroscopicity, and/or efficacy, so as to achieve good effects in drug preparation and drug use stages.

## Summary of the Invention

In order to solve the above technical problems, the present invention provides a crystal form A of a compound

represented by formula (I),

Chem 1 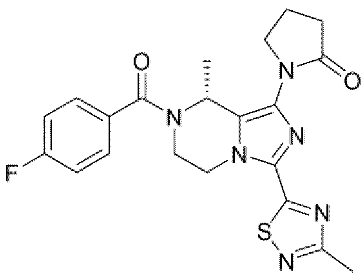 (I)

wherein X-ray powder diffraction of the crystal form A expressed as a 2θ angle using Cu-Kα radiation has characteristic peaks at 10.40±0.20°, 11.79±0.20°, 19.51±0.20°, and 20.81±0.20°.

Preferably, the X-ray powder diffraction of the crystal form A expressed as a 2θ angle using Cu-Kα radiation has characteristic peaks at 10.40±0.20°, 11.79±0.20°, 15.92±0.20°, 16.92±0.20°, 19.51±0.20°, 20.81±0.20°, 21.19 ±0.20°, and 22.91±0.20°.

In some embodiments, the X-ray powder diffraction of the crystal form A expressed as a 2θ angle using Cu-Kα radiation further has characteristic peaks at 3.17±0.20°, 8.50±0.20°, 13.24±0.20°, 15.15±0.20°, 19.03±0.20°, 25.03 ±0.20°, and/or 26.95±0.20°.

In some embodiments of the present invention, the above crystal form A has an XRPD pattern substantially as shown in FIG. 1.

In some embodiments of the present invention, analysis data of the XRPD pattern of the above crystal form A is as shown in Table 1:

Table 1

| Peak position [°2θ] | Relative intensity [%] |
|---|---|
| 3.1675 | 17.71 |
| 8.5029 | 18.72 |
| 10.4006 | 48.49 |
| 11.7914 | 100.00 |
| 13.2360 | 15.25 |
| 15.1538 | 16.62 |
| 15.9176 | 31.12 |
| 16.9234 | 36.18 |
| 19.0330 | 15.89 |
| 19.5112 | 64.15 |
| 20.8115 | 55.56 |
| 21.1910 | 35.01 |
| 22.9115 | 37.45 |
| 25.0305 | 18.89 |
| 26.9502 | 15.23 |

In some embodiments of the present invention, analysis data of the XRPD pattern of the above crystal form A is as shown in Table 2:

Table 2

| Serial No. | Peak position [°2θ] | Relative intensity [%] |
|---|---|---|
| 1 | 3.1675 | 17.71 |
| 2 | 8.5029 | 18.72 |
| 3 | 10.4006 | 48.49 |
| 4 | 11.7914 | 100.00 |
| 5 | 12.5303 | 14.20 |
| 6 | 13.2360 | 15.25 |

(continued)

| Serial No. | Peak position [°2θ] | Relative intensity [%] |
|---|---|---|
| 7 | 15.1538 | 16.62 |
| 8 | 15.9176 | 31.12 |
| 9 | 16.9234 | 36.18 |
| 10 | 17.9078 | 13.19 |
| 11 | 19.0330 | 15.89 |
| 12 | 19.5112 | 64.15 |
| 13 | 20.8115 | 55.56 |
| 14 | 21.1910 | 35.01 |
| 15 | 22.9115 | 37.45 |
| 16 | 24.8041 | 9.95 |
| 17 | 25.0305 | 18.89 |
| 18 | 25.5883 | 14.23 |
| 19 | 26.6367 | 12.48 |
| 20 | 26.9502 | 15.23 |
| 21 | 27.8344 | 5.28 |
| 22 | 28.3403 | 7.42 |
| 23 | 28.6564 | 9.72 |
| 24 | 29.9257 | 3.81 |
| 25 | 31.1271 | 6.84 |
| 26 | 33.0470 | 2.53 |
| 27 | 36.9777 | 3.67 |

In some embodiments of the present invention, the above crystal form A has weight loss of up to 0.244% at 150±3 °C.

In some embodiments of the present invention, the above crystal form A has a TGA pattern substantially as shown in FIG. 2.

In some embodiments of the present invention, the above crystal form A has a peak value of an endothermic peak at 272.53±3 °C.

In some embodiments of the present invention, the above crystal form A has a DSC pattern substantially as shown in FIG. 3.

The present invention further provides a method for preparing the crystal form A of the compound represented by formula (I), comprising preparation through an anti-solvent method, solvent volatilization method, cooling precipitation method, suspension stirring method, thermal cycling method, gas-solid penetration method, polymer induction method, or grinding method using the compound represented by formula (I) as a raw material.

Preferably, the method for preparing the crystal form A includes the following steps:

(a) adding the compound represented by formula (I) to a solvent to form a suspension; wherein the solvent is selected from an organic solvent, water, or a mixed solvent of an organic solvent and water;
(b) stirring the suspension at 25-60°C for 8-120 h; and
(c) centrifuging and drying the suspension (for example, drying for 8-16 h) after the step (b) is complete, to obtain the crystal form A.

In some embodiments of the present invention, the organic solvent may be selected from one or more of methanol, ethanol, isopropanol, acetone, methyl isobutyl ketone, heptane, toluene, m-xylene, dichloromethane, trichloromethane, anisole, methyl tert-butyl ether, dimethylacetamide, N-methylpyrrolidone, dimethyl sulfoxide, 2-methyltetrahydrofuran, 1,4-dioxane, acetonitrile, tetrahydrofuran, ethyl acetate, and isopropyl acetate.

The present invention further provides a pharmaceutical composition, comprising the above crystal form A.

According to an embodiment of the present invention, the pharmaceutical composition further comprises a pharmaceutically acceptable pharmaceutical adjuvant, for example, including but not limited to one or more than two of excipients, fillers, lubricants, binders, disintegrants, inorganic salts, solvents, dissolution aids, suspending agents, isotonic agents, buffers, preservatives, antioxidants, colorants, foaming agents, and flavoring agents.

According to an embodiment of the present invention, the pharmaceutical composition further comprises one or more active ingredients other than the above crystal form A.

In the pharmaceutical composition, dosages of the crystal form A and the one or more active ingredients other than the crystal form A are each a therapeutically effective amount.

The present invention further provides use of the above crystal form A or the pharmaceutical composition in the preparation of a pharmaceutical formulation, wherein the pharmaceutical formulation is a NK-3 receptor antagonist.

According to an embodiment of the present invention, the pharmaceutical formulation is used for preventing and/or treating a disease mediated by a NK-3 receptor, for example, is used for preventing and/or treating depression, anxiety disorder, psychosis, schizophrenia, psychotic disorder, bipolar disorder, cognitive disorder, Parkinson's disease, Alzheimer's disease, attention deficit hyperactivity disorder (ADHD), pain, convulsion, obesity, inflammatory disease, vomiting, preeclampsia, airway-related disease, dysgenesis, contraception and sex hormone-dependent disease, and/or gynecological disease-related disease.

According to an embodiment of the present invention, the sex hormone-dependent disease includes, but is not limited to, benign prostatic hyperplasia (BPH), prostatic hyperplasia, metastatic prostatic cancer, testicular cancer, breast cancer, ovarian cancer, androgen-dependent acne, male pattern alopecia, endometriosis, abnormal puberty, uterine fibrosis, uterine fibroma, hormone-dependent cancer, hyperandrogenism, hirsutism, masculinization, polycystic ovary syndrome (PCOS), premenstrual dysphoric disorder (PMDD), HAIR-AN syndrome (hyperandrogenism, insulin resistance, and acanthosis nigricans), hyperthecosis (HAIR-AN with luteinized theca cell proliferation in ovarian stroma), other manifestations of high intraovarian androgen concentrations (such as follicular maturation arrest, atresia, anovulation, dysmenorrhea, dysfunctional uterine bleeding, or infertility), androgen-producing tumor (virilizing ovarian tumor or adrenal tumor), hypermenorrhea, and/or adenomyosis.

According to an embodiment of the present invention, the airway -related disease includes, but is not limited to, chronic obstructive pulmonary disease, asthma, bronchial hyperresponsiveness, bronchoconstriction, and/or cough.

In some embodiments, the pharmaceutical formulation is used for treating and/or preventing menopausal syndrome-related disease, wherein the menopausal syndrome includes symptoms, such as hot flash, sweating, palpitation, dizziness, and/or obesity.

According to an embodiment of the present invention, the pharmaceutical formulation may be in a dosage form, such as powder, tablet (e.g., coated tablet, sustained-release or controlled-release tablet), lozenge, capsule (e.g., soft capsule or hard capsule), granule, pill, dispersible powder, suspension, solution, emulsion, elixir, syrup, aerosol, cream, ointment, gel, injection, lyophilized powder injection, or suppository.

According to an embodiment of the present invention, the pharmaceutical formulation may be administered by any one of: oral administration, buccal administration, sublingual administration, inhalation, topical application, or parenteral, intravenous, subcutaneous, acupointal or intramuscular injection, or rectal administration.

The present invention further provides a method for preventing and/or treating a disease, comprising administering to a patient a therapeutically effective amount of the crystal form A or the pharmaceutical composition;

the disease is a disease mediated by a NK-3 receptor, preferably depression, anxiety disorder, psychosis, schizophrenia, psychotic disorder, bipolar disorder, cognitive disorder, Parkinson's disease, Alzheimer's disease, attention deficit hyperactivity disorder, pain, convulsion, obesity, inflammatory disease, vomiting, preeclampsia, airway-related disease, dysgenesis, contraception and sex hormone-dependent disease, and/or gynecological disease-related disease;

or the disease is menopausal syndrome-related disease, wherein the menopausal syndrome includes symptoms, such as hot flash, sweating, palpitation, dizziness, and/or obesity.

Beneficial effects

The crystal form A provided in the present invention has stable properties and weak hygroscopicity, is suitable for prolonged storage, is suitable for preparing a drug, and can effectively inhibit NK3R.

Definitions and description of terms

Unless otherwise stated, the definitions of terms disclosed in the specification and claims of the present application include definitions as examples, example definitions, preferred definitions, definitions of specific compounds in the embodiments, etc., and may be combined with each other in any way. Such combinations should be encompassed within the scope disclosed in the specification of the present application.

The term "crystal form" refers to a crystal form that has same chemical composition but a different spatial arrangement of molecules and/or ions forming the crystal.

The compound represented by formula (I) is a "free base", and the "crystal form A of free base" is the "crystal form A of the compound represented by formula (I)".

The term "therapeutically effective amount" refers to an amount of the crystal Form A and the one or more active

ingredients other than the crystal form A of the present invention sufficient to achieve the intended use, including, but not limited to, disease treatment as defined below. A therapeutically effective amount may vary depending on the intended use (in vitro or in vivo), or the subject and disease condition being treated such as the weight and age of the subject, the severity of the disease condition and the mode of administration, etc., and can be easily determined by those of ordinary skills in the art. The specific dosage will vary depending on: the selected particular active ingredient, the administration regimen followed, whether it is administered in combination with other compounds, the time arrangement of administration, the tissue to which it is administered, and the physical delivery system on which it is carried.

The term "patient" refers to any animal including mammals, preferably mice, rats, other rodents, rabbits, dogs, cats, pigs, cattle, sheep, horses or primates, most preferably humans.

The term "plurality" means two or more, such as two or more than two.

The term "150±3°C" represents 147-153°C, which may be 147°C, 148°C, 149°C, 150°C, 151°C, 152°C, 153°C, or a value between any two thereof.

The term "272.53±3°C" represents 269.53-275.53°C, which may be 269.53°C, 270.00°C, 271.00°C, 272.00°C, 275.53°C, or a value between any two thereof.

## Description of Drawings

FIG. 1: XRPD pattern of crystal form A of a compound represented by formula (I);
FIG. 2: TGA pattern of crystal form A of a compound represented by formula (I);
FIG. 3: DSC pattern of crystal form A of a compound represented by formula (I);
FIG. 4: DVS pattern of crystal form A of a compound represented by formula (I);
FIG. 5: XRPD comparison diagram of crystal form A of a compound represented by formula (I) before and after DVS testing; and
FIG. 6: XRPD comparison diagram of crystal form A of a compound represented by formula (I) before and after stability evaluation.

## Detailed Description

The technical solution of the present invention will be further described in detail below in conjunction with specific embodiments. It should be understood that the following embodiments are only intended to exemplify and explain the present invention and should not be construed as limiting the scope of protection of the present invention. All technologies realized based on the above contents of the present invention are included in the scope that the present invention intends to protect.

Unless otherwise stated, the raw materials and reagents used in the following examples are commercially available, or may be prepared in accordance with known methods.

The following instruments, parameters, characterizations, and test methods are used in the examples:
(1) X-ray powder diffractometer (XRPD)

Model: PANalytical X-ray powder diffraction analyzer X'Pert3;
X-ray: Cu, kα, Kα1 (Å): 1.540598; Kα2 (Å): 1.544426; Kα2/Kα1 intensity ratio: 0.50;
X-ray tube setting: 45 kV, 40 mA;
Divergence slit: constant 1/8°;
Scan mode: continuous;
Scanning range (°2 Theta): 3-40;
Scan time per step (s): 46.7;
Scanning step length (°2 Theta): 0.0263; and
Test time (min): 5.

(2) Thermal Gravimetric Analyzer (TGA)

Instrument model: Discovery 5500;
Sample pan: open or sealed aluminum pan;
Initial temperature: room temperature (below 35°C);
Final temperature: suspending next section at 300 °C or when weight<80% (w/w) (weight loss of the compound does not exceed 20% (w/w));
Heating rate: 10 °C/min;

Nitrogen flow rate: stable at 10 mL/min; sample chamber 25 mL/min;
Sample amount: about 2-10 mg.

(3) Differential Scanning Calorimeter (DSC)

Instrument model: TA Discovery 2500 or Q2000;
Sample pan: Tzero pan and Tzero sealing cover, with a pin hole with a diameter of 0.7 mm;
Temperature range: 30-300°C or before decomposition;
Heating rate: 10 °C/min;
Nitrogen flow rate: 50 mL/min;
Sample amount: about 0.5-2 mg;

(4) Dynamic vapor sorption (DVS)

DVS curve is collected on DVS Intrinsic of SMS (Surface Measurement Systems) Relative humidity at 25 °C is calibrated based on deliquescent points of LiCl, $Mg(NO_3)_2$, and KCl.
Temperature: 25 °C;
Sample amount: 10-20 mg;
Protecting gas and flow rate: $N_2$, 200 mL/min;
dm/dt: 0.002%/min;
Minimum dm/dt equilibration time: 10 min;
Maximum equilibration time: 180 min;
RH range: 0%RH-95%RH-0%RH;
RH gradient: 10%RH (0%RH-90%RH & 90%RH-0%RH); 5%RH (90%RH-95%RH & 95%RH-90%RH)

(5) Ultra performance liquid chromatograph
Waters H-Class ultra performance liquid chromatograph is used to collect solubility and purity data of the items. Specific instruments and test parameters are shown in Table 3.

Table 3

| Parameters | Set values | |
|---|---|---|
| Chromatographic column | Waters Xbridge C18, 150*4.6 mm, 5 μm | |
| Mobile phase | A: 0.1% TFA in water | |
| | B: 0.1% TFA in acetonitrile | |
| Gradient | Time (min) | % mobile phase B |
| | 0.00 | 60 |
| | 6.00 | 60 |
| Time | 6.0 min | |
| Flow rate | 1.0 mL/min | |
| Injection volume | 5 μL | |
| Detection wavelength | UV at 327 nm | |
| Column temperature | 35°C | |
| Sample injector temperature | Room temperature | |
| Diluent | ACN/$H_2O$ (1:1, v/v) | |

Patent application PCT/CN2022/087947 discloses the compound represented by formula (I), and all contents involved in this patent application are incorporated herein by reference.
Comparison table of Chinese and English names of solvents selected in the experiments

Table 3

| English | Chinese | English | Chinese |
|---|---|---|---|
| MeOH | Methanol | Toluene | Methylbenzene |
| EtOH | Ethanol | Anisole | Phenyl methyl ether |
| IPA | Isopropanol | DCM | Dichloromethane |
| Acetone | Propanone | n-Heptane | Normal heptane |
| MEK | Methyl ethyl ketone | CPME | Cyclopentyl methyl ether |
| EtOAc | Ethyl acetate | DMAc | Dimethylacetamide |
| IPAc | Isopropyl acetate | Cyclohexane | Cyclohexane |
| MTBE | Methyl tert-butyl ether | n-BuOH | n-butanol |
| THF | Tetrahydrofuran | m-Xylene | meta-xylene |
| 2-MeTHF | 2-methyltetrahydrofuran | Methyl acetate | Methyl acetate |
| 1,4-Dioxane | 1,4-Dioxane | Isopropyl ether | Isopropyl ether |
| ACN | Acetonitrile | $H_2O$ | Water |
| Captex 200P | Propylene di(octanoate) | NMP | N-methyl-2-pyrrolidone |
| MIBK | Methyl isobutyl ketone | | |

Preparation Example Compound represented by formula (I)

The structures of the compounds in the present invention are determined by nuclear magnetic resonance (NMR) or/and liquid chromatography-mass spectrometry (LC-MS). NMR chemical shifts ($\delta$) are given in parts per million (ppm). The NMR measurement is performed using a Bruker AVANCE-400 nuclear magnetic resonance spectrometer, with solvents of deuterated dimethyl sulfoxide (DMSO-$d_6$), deuterated methanol ($CD_3OD$), and deuterated chloroform ($CDCl_3$), and with an internal standard of tetramethylsilane (TMS).

An Agilent 1200 Infinity Series mass spectrometer is used for liquid chromatography-mass spectrometry (LC-MS) measurements. The HPLC measurement is performed using an Agilent 1200DAD high pressure liquid chromatograph (Sunfire C18 150×4.6 mm chromatographic column) and a Waters 2695-2996 high pressure liquid chromatograph (Gimini C18 150×4.6 mm chromatographic column).

Yantai Huanghai HSGF254 or Qingdao GF254 silica gel plate is used as the silica gel plate for thin layer chromatography, the specification used for TLC is from 0.15 mm to 0.20 mm, and the specification of the thin layer chromatography for separation and purification of products is from 0.4 mm to 0.5 mm. Yantai Huanghai silica gel of 200-300 mesh is generally used as the carrier for column chromatography.

Unless otherwise specified, all reactions in the present invention are carried out under continuous magnetic stirring in a dry nitrogen or argon atmosphere in a dry solvent at a reaction temperature (°C).

1.1 Synthesis of intermediate 001

Preparation of (4-fluorophenyl)(8-methyl-3-(3-methyl-1,2,4-thiadiazol-5-yl)-5,6-dihydroimidazo[1,5-a]pyrazin-7(8H)-yl)methanone (001)

Chem 2

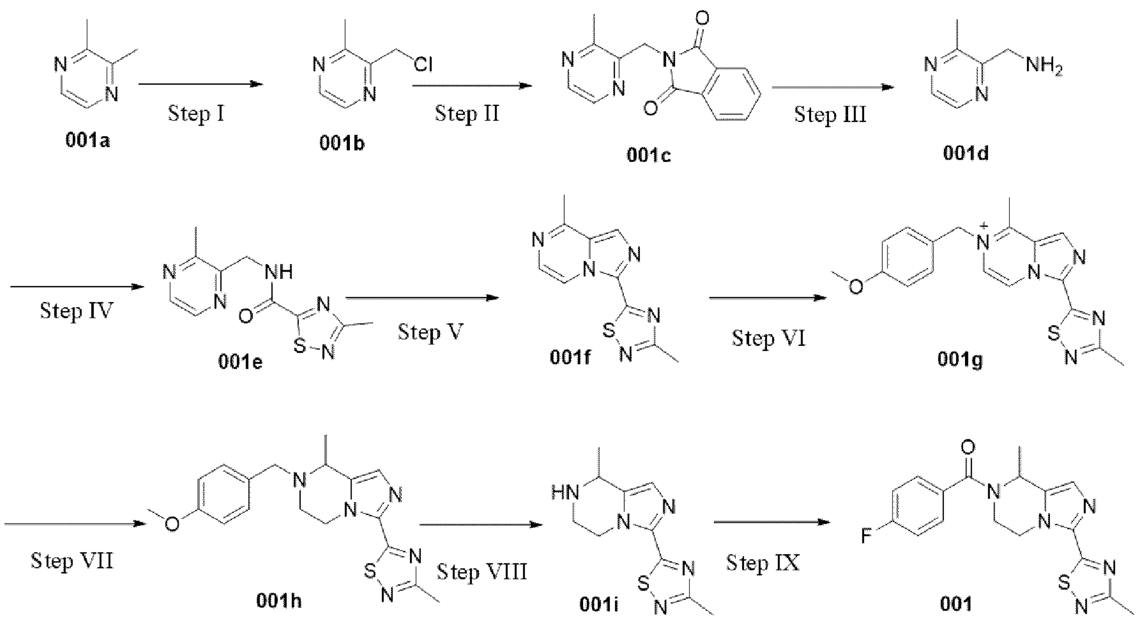


Step I: Preparation of 2-(chloromethyl)-3-methylpyrazine

2,3-dimethylpyrazine 001a (10 g, 92.47 mmol) was added to a solvent carbon tetrachloride (250 mL). N-chlorosuccinimide (14.83 g, 110.96 mmol) and benzoyl peroxide (224 mg, 9.25 mmol) were successively added. The reaction mixture was kept for reaction under the protection of nitrogen at 80 °C for 16 h. After the completion of the reaction was monitored by LCMS, the solvent was spin-dried. The mixture was extracted with water (150 mL) and dichloromethane (3×100 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=10:1) to provide the captioned product 2-(chloromethyl)-3-methylpyrazine 001b (3.20 g, colorless oil, yield: 22%).
MS m/z (ESI): 143.2$[M+1]^+$.
$^1$H NMR (400 MHz, $CDCl_3$) $\delta$ 8.45 (d, *J* = 2.0 Hz, 1H), 8.38 (d, *J* = 2.0 Hz, 1H), 4.71 (s, 2H), 2.69 (s, 3H).

Step II: Preparation of 2-((3-methylpyrazin-2-yl)methyl)isoindoline-1,3-dione

2-(chloromethyl)-3-methylpyrazine 001b (3.20 g, 22.44 mmol) was added to a solvent N,N-dimethylformamide, potassium phthalimide (6.23 g, 33.66 mmol) was added, and the reaction mixture was kept for reaction under the protection of nitrogen at 110 °C for 8 h. After the completion of the reaction was monitored by LCMS, the solvent was spin-dried. The mixture was extracted with water and ethyl acetate for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=1:1) to provide the captioned product 2-((3-methylpyrazin-2-yl)methyl)isoindoline-1,3-dione 001c (3.10 g, light yellow solid, yield: 95%).
MS m/z (ESI): 254.2[M+1]+.
$^1$H NMR (400 MHz, $CDCl_3$) $\delta$ 8.33 (d, *J*=2.4 Hz, 1H), 8.24 (d, *J*=2.4 Hz, 1H), 7.90 (dd, *J*=5.6, 3.2 Hz, 2H), 7.75 (dd, *J*=5.6, 3.2 Hz, 2H), 5.02 (s, 2H), 2.70 (s, 3H).

Step III: Preparation of 2-(aminomethyl)-3-methylpyrazine

2-((3-methylpyrazin-2-yl)methyl)isoindoline-1,3-dione 001c (2.00 g, 7.90 mmol) was added to a solvent ethanol (50 mL). Hydrazine hydrate (3.95 g, 79 mmol) was added. The reaction mixture was kept for reaction under the protection of nitrogen at 80 °C for 6 h. After the completion of the reaction was monitored by LCMS, the solvent was spin-dried, and water (150 mL) was added. The mixture was extracted with dichloromethane/methanol (1/1, V/V, 50 mL) for liquid separation. The organic phase was washed with a saturated sodium chloride solution, dried over anhydrous sodium sulfate, and suction filtered. The filtrate was concentrated under reduced pressure, to provide a crude product 2-(aminomethyl)-3-methylpyrazine 001d (300 mg, yellow oil, yield: 28%).
MS m/z (ESI): 124.3$[M+1]^+$.

$^1H$ NMR (400 MHz, $CDCl_3$) $\delta$ 8.36 (s, 1H), 8.33 (d, *J*=2.4 Hz, 1H), 4.02 (s, 2H), 2.54 (s, 3H).

Step IV: Preparation of 3-methyl-N-((3-methylpyrazin-2-yl)methyl)-1,2,4-thiadiazole-5-carboxamide

3-methyl-1,2,4-thiadiazole-5-carboxylic acid (280 mg,1.94 mmol) was added to a solvent dichloromethane (10 mL). 0.5 mL of oxalyl chloride (0.5 mL) and N,N-dimethylformamide (0.1 mL) were successively added. The reaction mixture was kept for reaction at 25 °C for 0.5 h. After the completion of the reaction was monitored by LCMS, the solvent was spin-dried to obtain a crude product 3-methyl-1,2,4-thiadiazole-5-carbonyl chloride.

2-(aminomethyl)-3-methylpyrazine 001d (200 mg, 1.62 mmol) and triethylamine (246 mg, 2.43 mmol) were added to a solvent dichloromethane (10 mL), to which the crude product 3-methyl-1,2,4-thiadiazole-5-carbonyl chloride dissolved in dichloromethane (5 mL) was slowly added dropwise. The reaction mixture was kept for reaction at 25 °C for 0.5 h. After the completion of the reaction was monitored by LCMS, the mixture was extracted with water (30 mL) and dichloromethane (3×20 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=1:1) to provide the captioned product 3-methyl-N-((3-methylpyrazin-2-yl)methyl)-1,2,4-thiadiazole-5-carboxamide 001e (170 mg, yellow solid, yield: 40%).

MS m/z (ESI): 250.2$[M+1]^+$.

$^1H$ NMR (400 MHz, $CDCl_3$) $\delta$ 8.59 (s, 1H), 8.46 (s, 2H), 4.78 (d, *J*=4.8 Hz, 2H), 2.76 (s, 3H), 2.65 (s, 3H).

Step V: Preparation of 3-methyl-5-(8-methylimidazo[1,5-a]pyrazin-3-yl)-1,2,4-thiadiazole

3-methyl-N-((3-methylpyrazin-2-yl)methyl)-1,2,4-thiadiazole-5-carboxamide 001e (400 mg, 1.60 mmol) was added to a solvent acetonitrile (10 mL). Phosphorus oxychloride (0.74 g, 4.80 mmol) and N,N-dimethylformamide (0.2 mL) were successively added. The reaction mixture was kept for reaction under the protection of nitrogen at 85 °C for 48 h. After the completion of the reaction was monitored by LCMS, the solvent was spin-dried. The mixture was extracted with a saturated sodium bicarbonate solution (50 mL) and ethyl acetate (3×20 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=1:1) to provide the captioned product 3-methyl-5-(8-methylimidazo[1,5-a]pyrazin-3-yl)-1,2,4-thiadiazole 001f (250 mg, yellow solid, yield: 60%).

MS m/z (ESI): 232.2$[M+1]^+$.

$^1H$ NMR (400 MHz, $CDCl_3$) $\delta$ 9.58 (d, *J*=3.2 Hz, 1H), 8.51 (s, 1H), 7.81 (s, 1H), 3.22 (s, 3H), 2.83 (s, 3H).

Step VI: Preparation of 7-(4-methoxybenzyl)-8-methyl-3-(3-methyl-1,2,4-thiadiazol-5-yl)imidazo[1,5-a]pyrazine-7-onium

3-methyl-5-(8-methylimidazo[1,5-a]pyrazin-3-yl)-1,2,4-thiadiazole 001f (1.0 g, 4.3 mmol) was added to a solvent acetonitrile (6 mL). Potassium iodide (357 mg, 2.15 mmol) and 1-(chloromethyl)-4-methoxybenzene (1.30 g, 8.60 mmol) were successively added. The reaction mixture was kept for reaction under the protection of nitrogen at 8 °C for 16 h. After the completion of the reaction was monitored by LCMS, the solvent was spin-dried to provide the captioned crude product 7-(4-methoxybenzyl)-8-methyl-3-(3-methyl-1,2,4-thiadiazol-5-yl)imidazo[1,5-a]pyrazine-7-onium 001g (600 mg, yellow solid, yield: 34%).

MS m/z (ESI): 352.2$[M+1]^+$.

Step VII: Preparation of 5-(7-(4-methoxybenzyl)-8-methyl-5,6,7,8-tetrahydroimidazo[1,5-a]pyrazin-3-yl)-3-methyl-1,2,4-thiadiazole

7-(4-methoxybenzyl)-8-methyl-3-(3-methyl-1,2,4-thiadiazol-5-yl)imidazo[1,5-a]pyrazine-7-onium 001g (600 mg, 1.7 mmol) was added to a solvent ethanol (10 mL). Acetic acid (0.1 mL) and sodium cyanoborohydride (320 mg, 5.1 mmol) were successively added. The reaction mixture was kept for reaction under the protection of nitrogen at 0 °C for 0.5 h. After the completion of the reaction was monitored by LCMS, the solvent was spin-dried. The mixture was extracted with water (50 mL) and dichloromethane (3×20 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=1:1) to provide the captioned product 5-(7-(4-methoxybenzyl)-8-methyl-5,6,7,8-tetrahydroimidazo[1,5-a]pyrazin-3-yl)-3-methyl-1,2,4-thiadiazole 001h (300 mg, yellow solid, yield: 24%).

MS m/z (ESI): 356.2$[M+1]^+$.

Step VIII: Preparation of 3-methyl-5-(8-methyl-5,6,7,8-tetraimidazo[1,5-a]pyrazin-3-yl)-1,2,4-thiadiazole

5-(7-(4-methoxybenzyl)-8-methyl-5,6,7,8-tetrahydroimidazo[1,5-a]pyrazin-3-yl)-3-methyl-1,2,4-thiadiazole 001h (200 mg, 0.56 mmol) was added to a solvent trifluoroacetic acid (3 mL). The reaction mixture was kept for reaction under the protection of nitrogen at 100 °C for 16 h. After the completion of the reaction was monitored by LCMS, the reaction mixture was cooled to room temperature. The solvent was spin-dried to obtain a crude product, which was purified through a reverse-phase column (acetonitrile/water=1:10) to provide the captioned product 3-methyl-5-(8-methyl-5,6,7,8-tetrahydroimidazo[1,5-a]pyrazin-3-yl)-1,2,4-thiadiazole 001i (120 mg, white solid, yield: 72%).
MS m/z (ESI): 236.2[M+1]$^+$.
HNMR:$^1$H NMR (400 MHz, DMSO-$_{d6}$) $\delta$ 9.47 (s, 1H), 7.32 (s, 1H), 4.95-4.88 (m, 1H), 4.70 (q, J=6.4 Hz, 1H), 4.45-4.35 (m, 1H), 3.86-3.79 (m, 1H), 3.60-3.51 (m, 1H), 2.66 (s, 3H), 1.63 (d, J=6.8 Hz, 3H).

Step IX: Preparation of (4-fluorophenyl)(8-methyl-3-(3-methyl-1,2,4-thiadiazol-5-yl)-5,6-dihydroimidazo[1,5-a]pyrazin-7(8H)-yl)methanone

3-methyl-5-(8-methyl-5,6,7,8-tetrahydroimidazo[1,5-a]pyrazin-3-yl)-1,2,4-thiadiazole 001i (100 mg, 0.42 mmol) was dissolved in dichloromethane (4 mL). Triethylamine (64 mg, 0.63 mmol) and p-fluorobenzoyl chloride (80 mg, 0.50 mmol) were successively added. The reaction mixture was kept for reaction at 25 °C for 2 h. After the reaction was complete, the mixture was extracted with water (20 mL) and dichloromethane (2×20 mL) for liquid separation, then washed with 20 mL of a saturated sodium chloride solution, dried over anhydrous sodium sulfate, and concentrated. The resulting residue was purified through a reverse-phase chromatographic column (acetonitrile/water=1:1) to provide 001 (10.20 mg, white solid, yield: 25%).
MS m/z (ESI): 358.0 [M+1]$^+$.
$^1$H NMR (400 MHz, $CDCl_3$) $\delta$ 7.47 (dd, *J*=8.6, 5.3 Hz, 2H), 7.16 (t, J=8.6 Hz, 2H), 7.07 (s, 1H), 5.71 (br s, 1H), 5.06 (dd, J=13.8, 2.4 Hz, 1H), 4.43-4.35(m, 1H), 4.24-4.17(m, 1H), 3.54 (t, J=12.7 Hz, 1H), 2.68 (s, 3H), 1.61 (d, J=6.8 Hz, 3H).

1.2 Separation of intermediate 001

Preparation of (R)-(4-fluorophenyl)(8-methyl-3-(3-methyl-1,2,4-thiadiazol-5-yl)-5,6-dihydroimidazo[1,5-a]pyrazin-7(8H)-yl)methanone (002) and (S)-(4-fluorophenyl)(8-methyl-3-(3-methyl-1,2,4-thiadiazol-5-yl)-5,6-dihydroimidazo [1,5-a]pyrazin-7(8H)-yl)methanone (003)

Chem 3

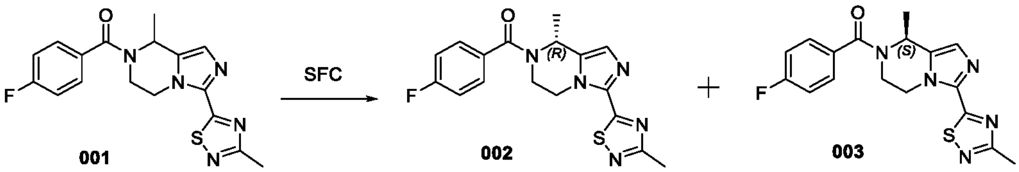


001 002 003

(4-fluorophenyl)(8-methyl-3-(3-methyl-1,2,4-thiadiazol-5-yl)-5,6-dihydroimidazo[1,5-a]pyrazin-7(8H)-yl) methanone 001 (100 mg) was separated by perp-SFC ($CO_2$/MeOH (0.2$NH_4$.OH)) to provide (R)-(4-fluorophenyl)(8-methyl-3-(3-methyl-1,2,4-thiadiazol-5-yl)-5,6-dihydroimidazo[1,5-a]pyrazin-7(8H)-yl)methanone (002) (39.20 mg, white solid) and (S)-(4-fluorophenyl)(8-methyl-3-(3-methyl-1,2,4-thiadiazol-5-yl)-5,6-dihydroimidazo[1,5-a]pyrazin-7(8H)-yl) methanone (003) (39.20 mg, white solid).
Corresponding data of the intermediate 002:

$$t_R=3.62\text{min}$$

HNMR:$^1$H NMR (400 MHz, $CDCl_3$) $\delta$ 7.47 (dd, J=8.6, 5.6 Hz, 2H), 7.16 (t, J=8.6 Hz, 2H), 7.06 (s, 1H), 5.93-5.52 (m, 1H), 5.06 (dd, J=13.8, 2.4 Hz, 1H), 4.54-4.11 (m, 2H), 3.54 (t, J=12.4 Hz, 1H), 2.68 (s, 3H), 1.61 (d, J=6.8 Hz, 3H).
Corresponding data of the intermediate 003:

$$t_R=1.82\text{ min}$$

MS m/z (ESI): 358.0 [M+1]$^+$.

HNMR:$^1$H NMR (400 MHz, $CDCl_3$) $\delta$ 7.51-7.43 (m, 2H), 7.16 (t, *J*=8.6 Hz, 2H), 7.06 (s, 1H), 5.91-5.47 (m, 1H), 5.05 (dd, *J*=13.8, 2.4 Hz, 1H), 4.52-4.06 (m, 2H), 3.54 (t, *J*=12.6 Hz, 1H), 2.67 (s, 3H), 1.61 (d, J=6.8 Hz, 3H).
SFC separation conditions:

Chromatographic column: Daicel CHIRALPAK OZ-H 250 mm*20 mm I.D., 5 μm
Mobile phase: $CO_2$/MeOH (0.2% NH4-OH)=70/30
Flow rate: 50 g/min.

1.3. Synthesis of a compound represented by formula (I)

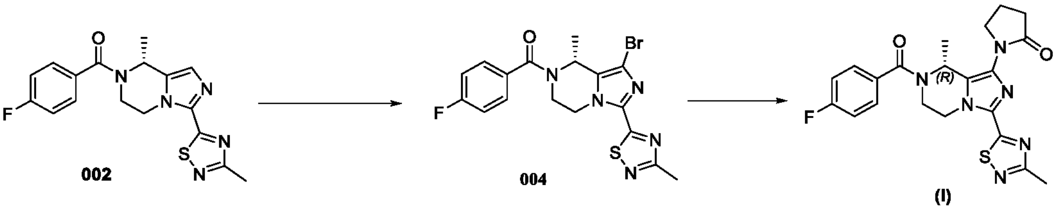

002 004 (I)

Chem 4

Step I: Preparation of (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone (004)

(R)-(4-fluorophenyl)(8-methyl-3-(3-methyl-1,2,4-thiadiazol-5-yl)-5,6-dihydroimidazo[1,5-a]pyrazin-7(8H)-yl) methanone 002 (50 mg, 0.013 mmol) was dissolved in dichloromethane (10 mL), and then N-bromosuccinimide (25 mg, 0.013 mmol) was added. The reaction mixture was stirred at room temperature for 1 h, extracted with dichloromethane (10 mL), washed with a saturated sodium chloride solution, dried over anhydrous sodium sulfate, and suction filtered. The filtrate was concentrated under reduced pressure, and the resulting residue was purified through a reverse-phase column (40% acetonitrile/water) to provide (R)-(1-bromo-8-methyl-3-(3-methyl-1,2,4-thiadiazol-5-yl)-5,6-dihydroimidazo[1,5-a] pyrazin-7(8H)-yl)(4-fluorophenyl)methanone 004 (16 mg, white solid, yield: 26%).
MS.m/z.(ESI):436.20$[M+1]^+$.
$^1$H NMR (400 MHz, $CDCl_3$) $\delta$ 7.49-7.45 (m, 2H), 7.19-7.17 (m, 2H), 6.02-5.81 (m, 1H), 5.13-4.92 (m, 2H), 4.25-4.13 (m, 1H), 3.68-3.49 (m, 1H), 2.68 (s, 3H), 1.64 (d, J=6.4 Hz, 3H).
HPLC: 254 nm (99.76%), 214 nm (99.53%)

Step II: Preparation of (R)-1-(7-(4-fluorobenzoyl)-8-methyl-3-(3-methyl-1,2,4-thiadiazol-5-yl)-5,6,7,8-tetrahydroimidazo [1,5-a]pyrazin-1-yl)pyrrolidin-2-one

(*R*)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 004 (40 mg, 0.09 mmol) was dissolved in 1,4-dioxane (2 mL). Then, pyrrolidin-2-one (100 mg, 0.19 mmol), potassium carbonate (38 mg, 0.28 mmol), cuprous iodide (1 mg, 0.005 mmol), 2-dicyclohexylphosphino-2',6'-diisopropoxy-1,1'-biphenyl (18 mg, 0.04 mmol), and (1R,2R)-N1,N2-dimethylcyclohexane-1,2-diamine (3 mg, 0.02 mmol) were successively added. The mixture was stirred while heating for reaction under the protection of nitrogen at 120 °C for 16 h. After the reaction was complete, the reaction was quenched with water. The mixture was extracted with ethyl acetate (3×10 mL). The organic phases were combined, and washed with saturated brine (20 mL). The organic phases were combined, dried over anhydrous sodium sulfate, and concentrated. The resulting residue was subjected to silica gel column chromatography (petroleum ether/ethyl acetate=50/50) to obtain a crude product, which was separated and purified through a reverse-phase column (mobile phase: acetonitrile/water=52/48) to provide (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one, i.e., the compound represented by formula (I) (6.53 mg, yield: 15%).
MS m/z (ESI): 441.1 $[M+1]^+$.
HPLC: 90.94% (214 nm), 97.04% (254 nm).
$^1$H NMR (400 MHz, $CDCl_3$) $\delta$ 7.56-7.45 (m, 2H), 7.21-7.12 (m, 2H), 5.99 (s, 1H), 5.11 (d, J=12.8 Hz, 1H), 5.01-4.72 (m, 1H), 4.28-4.12 (m, 2H), 3.64 (s, 1H), 3.43 (s, 1H), 2.69 (s, 3H), 2.52 (s, 2H), 2.30-2.12 (m, 2H), 1.36 (s, 3H).

Example 1: Preparation of crystal form A of a compound represented by formula (I)

Anti-solvent addition experiment

A total of 13 anti-solvent addition experiments were arranged using different solvents. About 15 mg of an initial sample of the compound represented by formula (I) was weighed in a 20 mL vial, and dissolved in a certain amount of a solvent. A corresponding anti-solvent was gradually added to the clear solution dropwise while stirring (500 rpm) until solid precipitation. In the case of no solid precipitation after 5 mL of the anti-solvent was added, the experiment was stopped. The clear solution was transferred to 5 °C/-20 °C, and stirred to induce solid precipitation. If the solution was still clear, the solution was transferred to room temperature for volatilization. Solid was collected, and subjected to XRPD testing. The result is as shown in Table 4. In the anti-solvent addition experiment, a crystal form A of the free base was obtained, but no other crystal forms were found.

Table 4 Summary of anti-solvent addition experiment

| Sample No. | Solvent | Anti-solvent | Result |
|---|---|---|---|
| A1 | $CHCl_3$ | IPA | Crystal form A of free base* |
| A2 | | EtOAc | Crystal form A of free base* |
| A3 | | MTBE | Crystal form A of free base |
| A4 | | 2-MeTHF | Crystal form A of free base* |
| A5 | | n-Heptane | Crystal form A of free base |
| A6 | DCM | EtOH | Crystal form A of free base* |
| A7 | | MIBK | Crystal form A of free base* |
| A8 | | IPAc | Crystal form A of free base# |
| A9 | | Toluene | Crystal form A of free base* |
| A10 | | Anisole | No solid precipitation* |
| A11 | THF | n-Heptane | Crystal form A of free base |
| A12 | | $H_2O$ | Crystal form A of free base |
| A13 | | MTBE | Crystal form A of free base# |
| *: No solid was obtained after the anti-solvent was added. After being transferred to 5 °C/-20 °C and stirred, the mixture was still clear, and was further transferred to room temperature for volatilization.<br>*: No solid was obtained after the anti-solvent was added. After the mixture was transferred to 5 °C and stirred, solid precipitated. | | | |

Slow volatilization experiment

A total of 5 slow volatilization experiments were arranged using different solvents. About 15 mg of an initial sample of the compound represented by formula (I) was weighed in a 3 mL vial, and was dissolved in an appropriate amount of a corresponding solvent. The solution was filtered through a PTFE filter membrane (0.45 μm) to obtain a clear solution. The solution was transferred to room temperature, sealed with a sealing film, which was punctured to make 4-5 small holes, and left to stand for natural volatilization. Solid was collected, and subjected to XRPD testing. The experimental result is as shown in Table 5. In the slow volatilization experiment, a crystal form A of the free base was obtained, but no other crystal forms were found.

Table 5 Summary of slow volatilization experiment

| Sample No. | Solvent, v:v | Result |
|---|---|---|
| B1 | $CHCl_3$ | Crystal form A of free base |
| B2 | THF | Crystal form A of free base |
| B3 | DCM | Crystal form A of free base |
| B4 | ACN | Crystal form A of free base |
| B5 | Acetone/DCM, 1:1 | Crystal form A of free base |

Slow cooling experiment

A total of 8 slow cooling experiments were arranged using different solvents. About 15 mg of an initial sample of the compound represented by formula (I) was weighed in a 3 mL vial, a corresponding solvent was added, the mixture was stirred at 50 °C for 2 h, and then the supernatant was filtered. The resulting filtrate was cooled from 50 °C to 5 °C at a rate of 0.1 °C/min, and was kept at a constant temperature of 5 °C. If the sample was a clear solution, the solution was transferred to -20 °C to induce solid precipitation. If the solution was still clear, the solution was transferred to room temperature for volatilization. The precipitated solid was collected, and subjected to XRPD testing. The experimental result is as shown in Table 6. In the slow cooling experiment, a crystal form A of the free base was obtained, but no other crystal forms were found.

Table 6 Summary of slow cooling experiment

| Sample No. | Solvent, v:v | Result |
|---|---|---|
| C1 | MeOH | Crystal form A of free base# |
| C2 | ACN | Crystal form A of free base# |
| C3 | DMAc | Crystal form A of free base* |
| C4 | NMP | Crystal form A of free base* |
| C5 | MIBK | Crystal form A of free base* |
| C6 | EtOAc | Crystal form A of free base* |
| C7 | 2-MeTHF | Crystal form A of free base* |
| C8 | $CHCl_3$/Toluene, 1:9 | Crystal form A of free base* |
| *: No solid was obtained after slow cooling. After being transferred to -20 °C, the mixture was still a clear solution, which was further transferred to room temperature for volatilization.<br>*: No solid was obtained after slow cooling. After the mixture was transferred to -20 °C, solid precipitated. | | |

Suspension stirring (room temperature/50 °C) experiment

A total of 38 suspension stirring experiments were arranged using different solvents at different temperatures. About 15 mg of an initial sample of the compound represented by formula (I) was weighed in a HPLC vial, and 1.0 mL of the solvents listed in Table 7 were added respectively. The resulting suspension was stirred at a corresponding temperature for a period of time, and centrifuged to collect solid for XRPD testing. If the sample was a clear solution, the solution was transferred to -20 °C and stirred to induce solid precipitation. If the solution was still clear, the solution was transferred to room temperature for volatilization. The precipitated solid was collected, and subjected to XRPD testing. The experimental result is as shown in Table 7. In the suspension stirring experiment, a crystal form A of the free base was obtained, but no other crystal forms were found.

Table 7 Summary of suspension stirring experiment

| Sample No. | Solvent, v:v | Temperature | Result |
|---|---|---|---|
| D1 | MeOH | Room temperature | Crystal form A of free base |
| D2 | Acetone | | Crystal form A of free base |
| D3 | 1,4-Dioxane | | Crystal form A of free base |
| D4 | ACN | | Crystal form A of free base |
| D5 | NMP | | Crystal form A of free base* |
| D6 | EtOH | | Crystal form A of free base |
| D7 | MIBK | | Crystal form A of free base |
| D8 | EtOAc | | Crystal form A of free base |
| D9 | MTBE | | Crystal form A of free base |
| D10 | 2-MeTHF | | Crystal form A of free base |
| D11 | n-Heptane | | Crystal form A of free base |
| D12 | $H_2O$ | | Crystal form A of free base |
| D13 | DMAc/$H_2O$, 1:4 | | Crystal form A of free base |
| D14 | THF/Toluene, 1:4 | | Crystal form A of free base |
| D15 | THF/IPA, 1:2 | | Crystal form A of free base |
| D16 | DCM/Anisole, 1:9 | | Crystal form A of free base |
| D17 | $CHCl_3$/IPAc, 1:9 | | Crystal form A of free base |
| D18 | $CHCl_3$/Toluene, 1:4 | | Crystal form A of free base |
| D19 | DMSO/$H_2O$, 1:4 | | Crystal form A of free base |
| D20 | Acetone/$H_2O$, 984:16, aw (water activity)=0.2 | | Crystal form A of free base |
| D21 | Acetone/$H_2O$, 948:52, aw=0.4 | | Crystal form A of free base |
| D22 | Acetone/$H_2O$, 857:143, aw=0.6 | | Crystal form A of free base |
| D23 | Acetone/$H_2O$, 604:396, aw=0.8 | | Crystal form A of free base |
| D24 | DMAc/$H_2O$, 1:4 | | Crystal form A of free base |

(continued)

| Sample No. | Solvent, v:v | Temperature | Result |
|---|---|---|---|
| E1 | EtOH | 50°C | Crystal form A of free base |
| E2 | IPA | | Crystal form A of free base |
| E3 | MEK | | Crystal form A of free base |
| E4 | IPAc | | Crystal form A of free base |
| E5 | Anisole | | Crystal form A of free base* |
| E6 | 2-MeTHF | | Crystal form A of free base |
| E7 | Toluene | | Crystal form A of free base |
| E8 | $H_2O$ | | Crystal form A of free base |
| E9 | m-xylene | | Crystal form A of free base |
| E10 | THF/$H_2O$, 1:4 | | Crystal form A of free base |
| E11 | ACN/$H_2O$, 1:9 | | Crystal form A of free base |
| E12 | DMSO/Toluene, 1:9 | | Crystal form A of free base |
| E13 | $CHCl_3$/n-Heptane, 1:9 | | Crystal form A of free base |
| E14 | 1,4-Dioxane/Anisole, 1:4 | | Crystal form A of free base |
| *: After suspension stirring at room temperature or 50°C, the solid was fully dissolved. After being transferred to -20 °C and stirred, the mixture was still a clear solution, which was further transferred to room temperature for volatilization. | | | |

Thermal cycling experiment

A total of 12 thermal cycling experiments were arranged using different solvents. About 15 mg of an initial sample of the compound represented by formula (I) was weighed in a HPLC vial, and an appropriate amount of a solvent was added. The suspension was transferred into a biochemical incubator at 50 °C for a cyclic heating-cooling experiment (program: the suspension was kept at a constant temperature of 50 °C for 2 h, cooled to 5 °C at a rate of 0.1 °C/min, kept at a constant temperature of 5 °C for 2 h, and then heated to 50 °C at a rate of 0.1 °C/min. A total of 2 cycles were performed. Finally, all samples were kept at 5 °C). After the cyclic heating-cooling program was complete, the resulting solid was characterized by XRPD. If the sample was a clear solution, the solution was transferred to -20 °C and stirred to induce solid precipitation. If the solution was still clear, the solution was transferred to room temperature for volatilization. The experimental result is as shown in Table 8. In the thermal cycling experiment, a crystal form A of the free base was obtained, but no other crystal forms were found.

Table 8 Summary of thermal cycling experiment

| Sample No. | Solvent, v:v | Result |
|---|---|---|
| F1 | IPA | Crystal form A of free base |
| F2 | MIBK | Crystal form A of free base |
| F3 | n-Heptane | Crystal form A of free base |
| F4 | $H_2O$ | Crystal form A of free base |
| F5 | ACN | Crystal form A of free base |
| F6 | DMAc | Crystal form A of free base* |
| F7 | CPME | Crystal form A of free base |
| F8 | EtOH/$H_2O$, 1:4 | Crystal form A of free base |
| F9 | DMF/$H_2O$, 1:9 | Crystal form A of free base |
| F10 | MEK/Toluene, 1:4 | Crystal form A of free base |
| F11 | THF/n-Heptane, 1:9 | Crystal form A of free base |

(continued)

| Sample No. | Solvent, v:v | Result |
|---|---|---|
| F12 | IPAc/Anisole, 1:1 | Crystal form A of free base |
| *: After cyclic heating-cooling and stirring, the solid was fully dissolved. After being transferred to -20 °C and stirred, the mixture was still a clear solution, which was further transferred to room temperature for volatilization. | | |

Gas-solid penetration experiment

A total of 13 gas-solid penetration experiments were arranged in different solvents. About 15 mg of an initial sample of the compound represented by formula (I) was weighed in a 3 mL vial, a corresponding solvent was added to another 20 mL vial, and the 3 mL vial that was kept open was transferred into the 20 mL vial, which was sealed and left to stand at room temperature for a week. Then, solid was collected, and subjected to XRPD testing. If the solid was fully dissolved, the solid was transferred to room temperature for volatilization. The experimental result is as shown in Table 9. In the gas-solid penetration experiment, a crystal form A of the free base was obtained, but no other crystal forms were found.

Table 9 Summary of gas-solid penetration experiment

| Sample No. | Solvent | Result |
|---|---|---|
| G1 | DCM | Crystal form A of free base* |
| G2 | EtOH | Crystal form A of free base |
| G3 | MeOH | Crystal form A of free base |
| G4 | ACN | Crystal form A of free base |
| G5 | THF | Crystal form A of free base |
| G6 | Acetone | Crystal form A of free base |
| G7 | EtOAc | Crystal form A of free base |
| G8 | DMSO | Crystal form A of free base |
| G9 | $H_2O$ | Crystal form A of free base |
| G10 | 22.5 RH %, saturated potassium acetate solution | Crystal form A of free base |
| G11 | 43.2 RH %, saturated potassium carbonate solution | Crystal form A of free base |
| G12 | 57.6 RH %, saturated potassium bromide solution | Crystal form A of free base |
| G13 | 84.2 RH %, saturated potassium chloride solution | Crystal form A of free base |
| *: After one week of gas-solid penetration, the solid was fully dissolved, and the mixture was transferred to room temperature for volatilization. | | |

Polymer induction experiment

A total of 6 polymer induction experiments were arranged using different solvents. About 15 mg of an initial sample of the compound represented by formula (I) was weighed in a 3 mL vial, and dissolved in a certain amount of a solvent, to obtain a clear solution. If the solution was still not clear after 1.5 mL of the solvent was added, the solution was filtered through a PTFE filter membrane (0.45 μm) to obtain a clear solution. About 2 mg of a corresponding polymer was weighed, and added to the corresponding 3 mL vial. The vial was sealed with a sealing film at room temperature, which was punctured to make 4 small holes, and then transferred into a fume hood for natural volatilization. Solid was collected, and subjected to XRPD testing. The result is as shown in Table 10. In the polymer induction experiment, a crystal form A of the free base was obtained, but no other crystal forms were found.

Table 10 Summary of polymer induction experiment

| Sample No. | Solvent | Conditions | Result |
|---|---|---|---|
| H1 | $CHCl_3$ | Mixed polymer A | Crystal form A of free base |
| H2 | DCM | | Crystal form A of free base |
| H3 | THF | | Crystal form A of free base |
| H4 | $CHCl_3$ | Mixed polymer B | Crystal form A of free base |
| H5 | DCM | | Crystal form A of free base |
| H6 | THF | | Crystal form A of free base |
| Mixed polymer A: polyvinylpyrrolidone, polyvinyl alcohol, polyvinyl chloride, polyvinyl acetate, hydroxypropyl methylcellulose, and methylcellulose (mixed in equal masses); and mixed polymer B: polycaprolactone, polyethylene glycol, polymethyl methacrylate, sodium alginate, and hydroxyethyl cellulose (mixed in equal masses). | | | |

Grinding experiment

A total of 5 grinding experiments were arranged using different solvents. About 15 mg of an initial sample of the compound represented by formula (I) was weighed in a mortar, an appropriate amount of a corresponding solvent was not added or was added to the mortar, and the solid was manually ground (for about 5 min). After the grinding was complete, the resulting solid sample was subjected to XRPD testing. The experimental result is as shown in Table 11. In each of the grinding experiments, a crystal form A of the free base was obtained.

Table 11 Summary of grinding experiment

| Sample No. | Solvent | Result |
|---|---|---|
| I1 | Not added | Crystal form A of free base |
| I2 | $H_2O$ | Crystal form A of free base |
| I3 | EtOH | Crystal form A of free base |
| I4 | Acetone | Crystal form A of free base |
| I5 | EtOAc | Crystal form A of free base |

Example 2 Characterization of crystal form A of a compound represented by formula (I)

An XRPD pattern of the crystal form A of the free base prepared in Example 1 is as shown in FIG. 1, and analysis data thereof is as shown in Table 12.

Table 12 Analysis data of XRPD pattern of crystal form A of the free base of the compound represented by formula (I)

| No. | Pos. [°2θ] | Height [cts] | FWHM Left [°2θ] | d-spacing [Å] | Rel. Int. [%] |
|---|---|---|---|---|---|
| 1 | 3.1675 | 252.73 | 0.3070 | 27.89 | 17.71 |
| 2 | 8.5029 | 267.14 | 0.0768 | 10.40 | 18.72 |
| 3 | 10.4006 | 691.90 | 0.0768 | 8.51 | 48.49 |
| 4 | 11.7914 | 1426.97 | 0.0768 | 7.51 | 100.00 |
| 5 | 12.5303 | 202.67 | 0.1023 | 7.06 | 14.20 |
| 6 | 13.2360 | 217.61 | 0.0768 | 6.69 | 15.25 |
| 7 | 15.1538 | 237.19 | 0.1023 | 5.85 | 16.62 |
| 8 | 15.9176 | 444.02 | 0.0768 | 5.57 | 31.12 |
| 9 | 16.9234 | 516.24 | 0.0768 | 5.24 | 36.18 |
| 10 | 17.9078 | 188.27 | 0.0768 | 4.95 | 13.19 |

(continued)

| No. | Pos. [°2θ] | Height [cts] | FWHM Left [°2θ] | d-spacing [Å] | Rel. Int. [%] |
|---|---|---|---|---|---|
| 11 | 19.0330 | 226.78 | 0.1023 | 4.66 | 15.89 |
| 12 | 19.5112 | 915.34 | 0.1279 | 4.55 | 64.15 |
| 13 | 20.8115 | 792.86 | 0.1535 | 4.27 | 55.56 |
| 14 | 21.1910 | 499.56 | 0.1023 | 4.19 | 35.01 |
| 15 | 22.9115 | 534.38 | 0.1023 | 3.88 | 37.45 |
| 16 | 24.8041 | 141.96 | 0.1023 | 3.59 | 9.95 |
| 17 | 25.0305 | 269.52 | 0.1023 | 3.56 | 18.89 |
| 18 | 25.5883 | 203.11 | 0.1279 | 3.48 | 14.23 |
| 19 | 26.6367 | 178.05 | 0.0768 | 3.35 | 12.48 |
| 20 | 26.9502 | 217.34 | 0.1023 | 3.31 | 15.23 |
| 21 | 27.8344 | 75.27 | 0.1535 | 3.21 | 5.28 |
| 22 | 28.3403 | 105.92 | 0.1535 | 3.15 | 7.42 |
| 23 | 28.6564 | 138.77 | 0.1279 | 3.12 | 9.72 |
| 24 | 29.9257 | 54.38 | 0.3070 | 2.99 | 3.81 |
| 25 | 31.1271 | 97.63 | 0.1535 | 2.87 | 6.84 |
| 26 | 33.0470 | 36.06 | 0.8187 | 2.71 | 2.53 |
| 27 | 36.9777 | 52.35 | 0.3070 | 2.43 | 3.67 |

A TGA pattern of the crystal form A of the free base is as shown in FIG. 2, wherein the weight loss is up to 0.244% at 150 °C.

A DSC pattern of the crystal form A of the free base is as shown in FIG. 3, wherein a peak temperature of an endothermic peak is at 272.53 °C.

The crystal form A of the free base was subjected to DVS testing at a constant temperature of 25 °C to evaluate its hygroscopicity. The DVS result is as shown in FIG. 4. The moisture adsorption of the crystal form A of the free base is about 0.15% at 25 °C/80%RH, indicating that the crystal form A of the free base is almost non-hygroscopic. The XRPD results before and after the DVS testing are as shown in FIG. 5, indicating that the crystal form A of the free base did not change in the crystal form before and after the DVS testing.

The solid-state stability of the crystal form A of the free base was evaluated. An appropriate amount of sample was weighed, kept open at 60 °C for 1 day, as well as kept open at 25 °C/60%RH and kept open at 40 °C/75%RH for 1 week and 4 weeks respectively. Then, the sample was characterized by XRPD and HPLC to detect changes in the crystal form and chemical purity. The test results are summarized in Table 13, and the XRPD comparison diagram is as shown in FIG. 6. The result shows that after the crystal form A of the free base was kept open at 60 °C for 1 day, at 25 °C/60%RH and at 40 °C/75%RH for 1 week and 4 weeks respectively, the purity did not change significantly (<0.2%, area%), nor did the crystal form change, indicating that the crystal form A of the free base has good physicochemical stability under the current evaluation conditions.

Table 13 Stability test result of crystal form A of the free base of the compound represented by formula (I)

| Crystal form | Initial purity (area%) | Conditions (open) | HPLC purity | | Final crystal form |
|---|---|---|---|---|---|
| | | | Area% | Relative purity% | |
| Crystal form A of free base | 99.56 | 60 °C, 1 day | 99.49 | 99.9 | Crystal form A of free base |
| | | 25 °C/60%RH, 1 week | 99.48 | 99.9 | Crystal form A of free base |
| | | 25 °C/60%RH, 4 weeks | 99.45 | 99.9 | Crystal form A of free base |
| | | 40 °C/75%RH, 1 week | 99.49 | 99.9 | Crystal form A of free base |
| | | 40 °C/75%RH, 4 weeks | 99.49 | 99.9 | Crystal form A of free base |

Example 3 Biological evaluation of a compound represented by formula (I)

Determination of activity of the compound represented by formula (I) on human NK-3 receptor

This method is used to determine agonistic effects of the compound represented by formula (I) on the activity of human NK-3 receptor proteins expressed in stably transfected cell strains of human NK-3R/HEK293.

1. Experimental Materials and Instruments

1.1 Culture medium

F12 (Gibco, Cat#11765-047);

FBS (Corning, Cat# 35-076-CV);

Geneticin (Invitrogen, Cat# 10131); and

Penicillin/Streptomycin (Invitrogen, Cat# 15140).

1.2 Reagents

Fluo-4 Direct (Invitrogen, Cat# F10471);

HBSS (Gibco, Cat#14025076);

HEPES (Gibco, Cat#15630080); and

Bonine Serum Albumin (Sgima, Cat#B2064-100G).

1.3. Consumables of instruments

384 well Poly-D-Lysine protein coating plate (Greiner, Cat#781946);

FLIPR (Molecular Devices);

Vi-cell XR Cell Viability Analyzer (Beckman Coulter); and

Incubator (Thermo).

2. Experimental Steps

2.1 The stably transfected cell strains of human NK-3R/HEK293 were inoculated in a 384-well cell culture plate at an inoculation density of 12,000 cells/well/25 μL, and cultured under 5% $CO_2$ at 37 °C overnight;
2.2 20× Component A was thawed to room temperature, diluted with Assay Buffer to 2× working concentration, and kept at room temperature;
2.3 The cell culture plate was equilibrated at room temperature for 10 min, the culture medium was removed, and 20 μL of Assay Buffer and 20 μL of 2× Component A (200 g) were added, followed by centrifugation at room temperature for 3-5 sec, and incubation at 37 °C for 2 h;
2.4 The compound was diluted 3 times with DMSO in 384 PP_DMSO plate, and then 240 nl/well of the mixture was transferred into a working plate with Echo 550. 200 g of the mixture was kept at room temperature for 1 min. 40 μl of Assay Buffer was added into the working plate. 200 g of the resulting mixture was kept at room temperature for 1 min, and then fully mixed in an oscillator at 2,500 rpm for 20 min. Then, 200 g of the resulting mixture was kept at room temperature for 1 min;
2.5 2.5 nM of Neurokinin B TFA (6X) was prepared from the Assay Buffer, and 50 μL of the mixture was transferred onto 3657 plate;
2.6 The cell culture plate was taken out and left to stand at room temperature for 10 min, then 10 μL of the compound diluted in the step 2.4 was added into corresponding wells, and the cell culture plate was left to stand at 25 °C for 30 min; and
2.7 10 μL of the compound diluted in the step 2.5 was add into the corresponding experimental wells with FLIPR Tetra, and data was collected.

The antagonistic activity of the compound represented by formula (I) on the human NK-3 receptor was determined through the above experiments, to obtain an inhibition curve of the compound represented by formula (I) and determine concentrations ($IC_{50}$) of the corresponding compounds that inhibit 50% of the reference agonist. The specific $IC_{50}$ values are shown in Table 14.

Table 14: $IC_{50}$ value of antagonistic activity of the compound represented by formula (I) on human NK-3 receptor

| Compound No. | $IC_{50}$ (nM) |
|---|---|
| Fezolinetant | 734.3 |
| Compound represented by formula (I) | 12.1 |

Experimental conclusions:

The above data shows that the compound represented by formula (I) is a potent NK-3 receptor antagonist.

The embodiments of the present invention are described above. However, the present invention is not limited to the above embodiments. Any modification, equivalent replacement, improvement, or the like made within the spirit and principle of the present invention should be encompassed within the scope of protection of the present invention.

## Claims

**1.** A crystal form A of a compound represented by formula (I),

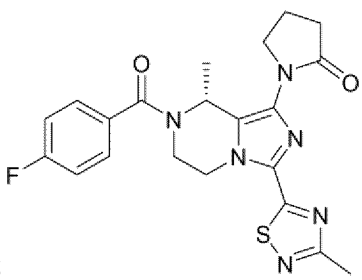

Chem 5 (I)

wherein X-ray powder diffraction of the crystal form A expressed as a 2θ angle using Cu-Kα radiation has characteristic peaks at 10.40±0.20°, 11.79±0.20°, 19.51±0.20°, and 20.81±0.20°.

**2.** The crystal form A according to claim 1, wherein the X-ray powder diffraction of the crystal form A expressed as a 20 angle using Cu-Kα radiation has characteristic peaks at 10.40±0.20°, 11.79±0.20°, 15.92±0.20°, 16.92±0.20°, 19.51±0.20°, 20.81±0.20°, 21.19±0.20°, and 22.91±0.20°, and
preferably, the X-ray powder diffraction of the crystal form A expressed as a 2θ angle using Cu-Kα radiation further has characteristic peaks at 3.17±0.20°, 8.50±0.20°, 13.24±0.20°, 15.15±0.20°, 19.03±0.20°, 25.03±0.20°, and/or 26.95±0.20°.

**3.** The crystal form A according to claim 1, wherein the crystal form A has an XRPD pattern substantially as shown in FIG. 1; and
preferably, analysis data of the XRPD pattern of the crystal form A is as shown in Table 1:

Table 1

| Peak position [°2θ] | Relative intensity [%] |
|---|---|
| 3.1675 | 17.71 |
| 8.5029 | 18.72 |
| 10.4006 | 48.49 |
| 11.7914 | 100.00 |
| 13.2360 | 15.25 |
| 15.1538 | 16.62 |

(continued)

| Peak position [°2θ] | Relative intensity [%] |
|---|---|
| 15.9176 | 31.12 |
| 16.9234 | 36.18 |
| 19.0330 | 15.89 |
| 19.5112 | 64.15 |
| 20.8115 | 55.56 |
| 21.1910 | 35.01 |
| 22.9115 | 37.45 |
| 25.0305 | 18.89 |
| 26.9502 | 15.23 |

**4.** A method for preparing the crystal form A of the compound represented by formula (I) according to any one of claims 1-3, comprising preparation through an anti-solvent method, solvent volatilization method, cooling precipitation method, suspension stirring method, thermal cycling method, gas-solid penetration method, polymer induction method, or grinding method using the compound represented by formula (I) as a raw material.

**5.** The preparation method according to claim 4, wherein the method for preparing the crystal form A includes steps of:

(a) adding the compound represented by formula (I) to a solvent to form a suspension; wherein the solvent is selected from an organic solvent, water, or a mixed solvent of an organic solvent and water;
(b) stirring the suspension at 25-60°C for 8-120 h; and
(c) centrifuging and drying the suspension after the step (b) is complete, to obtain the crystal form A; and

preferably, the organic solvent is selected from one or more of methanol, ethanol, isopropanol, acetone, methyl isobutyl ketone, heptane, toluene, m-xylene, dichloromethane, trichloromethane, anisole, methyl tert-butyl ether, dimethylacetamide, N-methylpyrrolidone, dimethyl sulfoxide, 2-methyltetrahydrofuran, 1,4-dioxane, acetonitrile, tetrahydrofuran, ethyl acetate, and isopropyl acetate.

**6.** A pharmaceutical composition, comprising the crystal form A according to any one of claims 1-3.

**7.** The pharmaceutical composition according to claim 6, wherein the pharmaceutical composition further comprises a pharmaceutically acceptable pharmaceutical adjuvant;

preferably, the pharmaceutical composition further comprises one or more active ingredients other than the above crystal form A; and
preferably, dosages of the crystal form A and the one or more active ingredients other than the crystal form A are each a therapeutically effective amount.

**8.** Use of the crystal form A according to any one of claims 1-3 or the pharmaceutical composition according to any one of claims 6-7 in the preparation of a pharmaceutical formulation.

**9.** The use according to claim 8, wherein the pharmaceutical formulation is used for preventing and/or treating a disease mediated by a NK-3 receptor;

is preferably used for preventing and/or treating depression, anxiety disorder, psychosis, schizophrenia, psychotic disorder, bipolar disorder, cognitive disorder, Parkinson's disease, Alzheimer's disease, attention deficit hyperactivity disorder, pain, convulsion, obesity, inflammatory disease, vomiting, preeclampsia, airway -related disease, dysgenesis, contraception and sex hormone-dependent disease, and/or gynecological disease-related disease;
or is used for treating and/or preventing menopausal syndrome-related disease, wherein the menopausal syndrome includes symptoms, such as hot flash, sweating, palpitation, dizziness, and/or obesity;
and is preferably a NK-3 receptor antagonist.

**10.** A method for preventing and/or treating a disease, comprising administering to a patient a therapeutically effective amount of the crystal form A according to any one of claims 1-3 or the pharmaceutical composition according to any

one of claims 6-7;

the disease is a disease mediated by a NK-3 receptor, preferably depression, anxiety disorder, psychosis, schizophrenia, psychotic disorder, bipolar disorder, cognitive disorder, Parkinson's disease, Alzheimer's disease, attention deficit hyperactivity disorder, pain, convulsion, obesity, inflammatory disease, vomiting, preeclampsia, airway-related disease, dysgenesis, contraception and sex hormone-dependent disease, and/or gynecological disease-related disease;
or the disease is menopausal syndrome-related disease, wherein the menopausal syndrome includes symptoms, such as hot flash, sweating, palpitation, dizziness, and/or obesity.

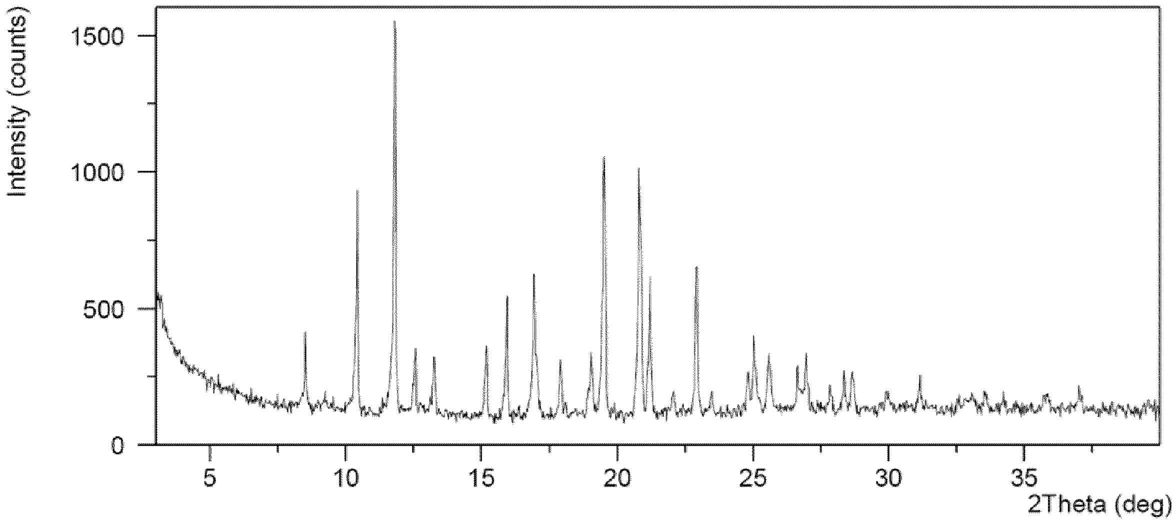
Intensity (counts)
1500
1000
500
0
5
10
15
20
25
30
35
2Theta (deg)

**FIG. 1**

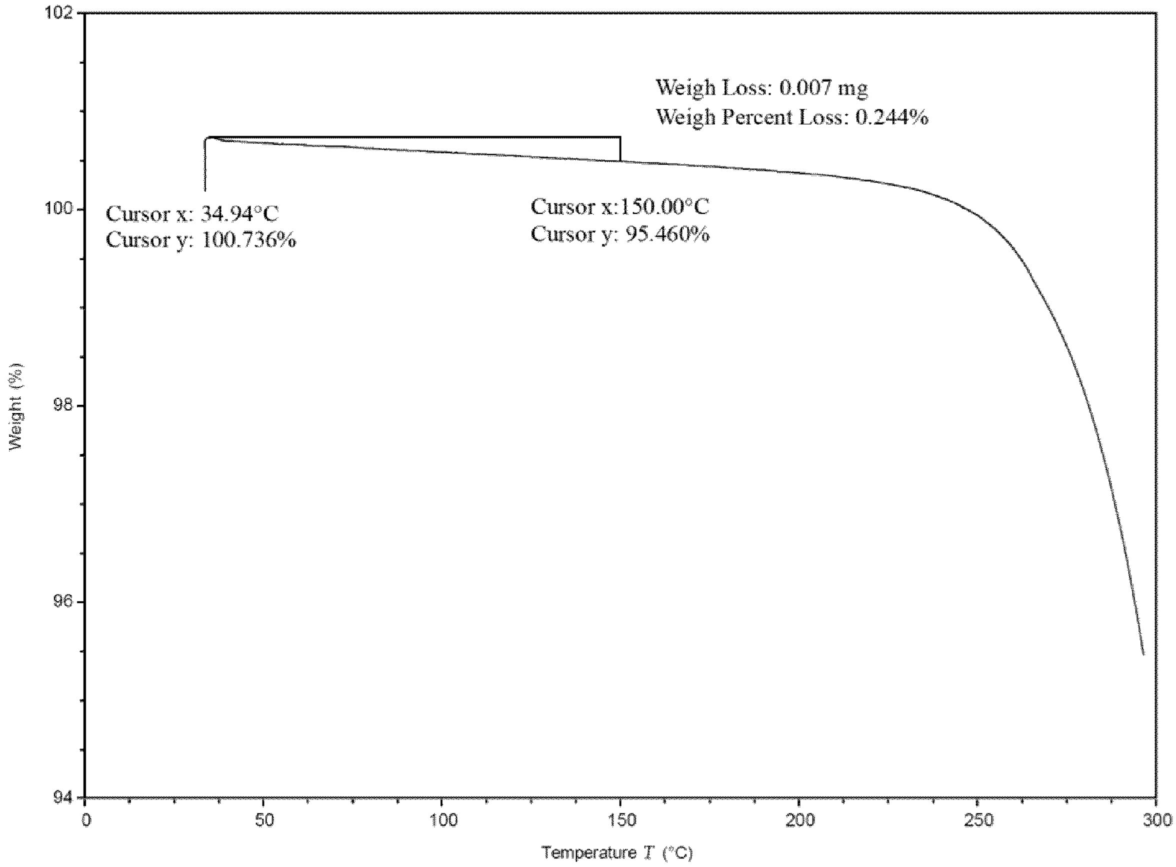
Weigh Loss: 0.007 mg
Weigh Percent Loss: 0.244%
Cursor x: 34.94°C
Cursor y: 100.736%
Cursor x:150.00°C
Cursor y: 95.460%
Weight (%)
102
100
98
96
94
0
50
100
150
200
250
300
Temperature T (°C)

**FIG. 2**

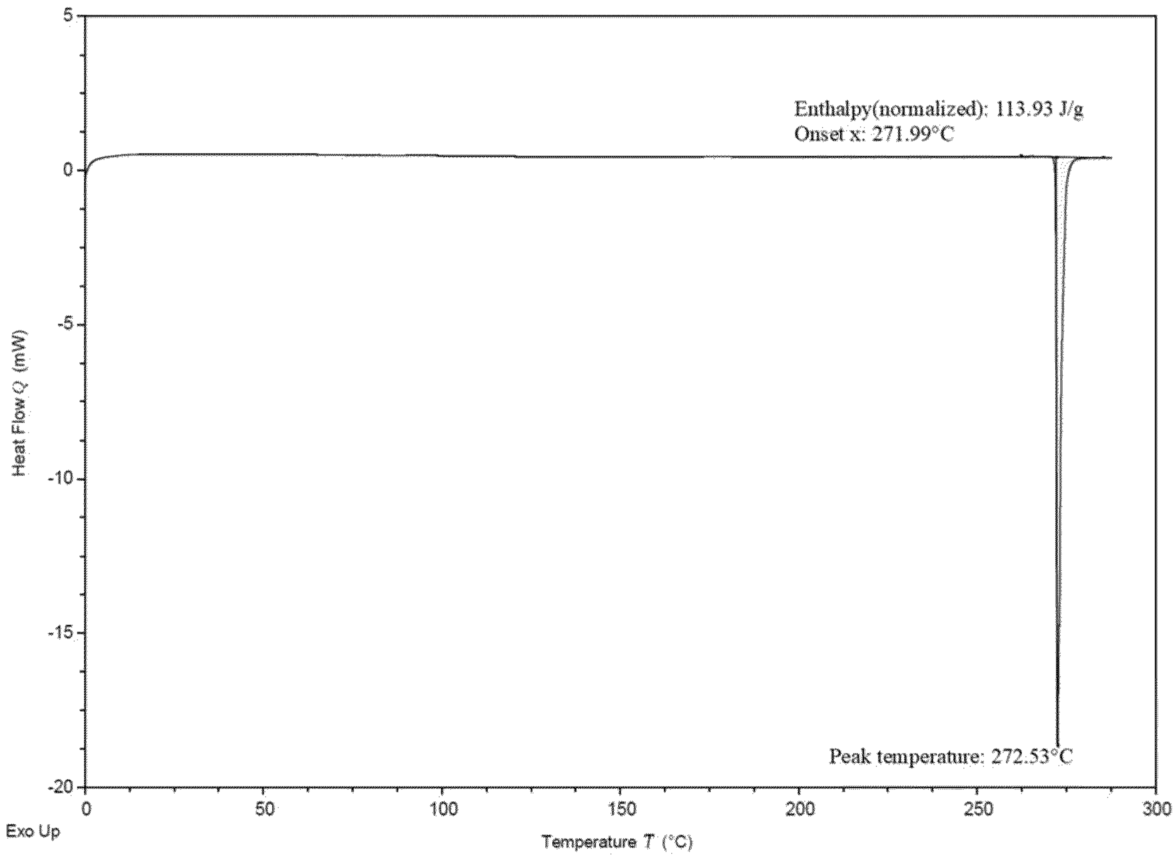

5
0
-5
-10
-15
-20
Heat Flow Q (mW)
Enthalpy(normalized): 113.93 J/g
Onset x: 271.99°C
Peak temperature: 272.53°C
0
50
100
150
200
250
300
Exo Up
Temperature T (°C)


FIG. 3

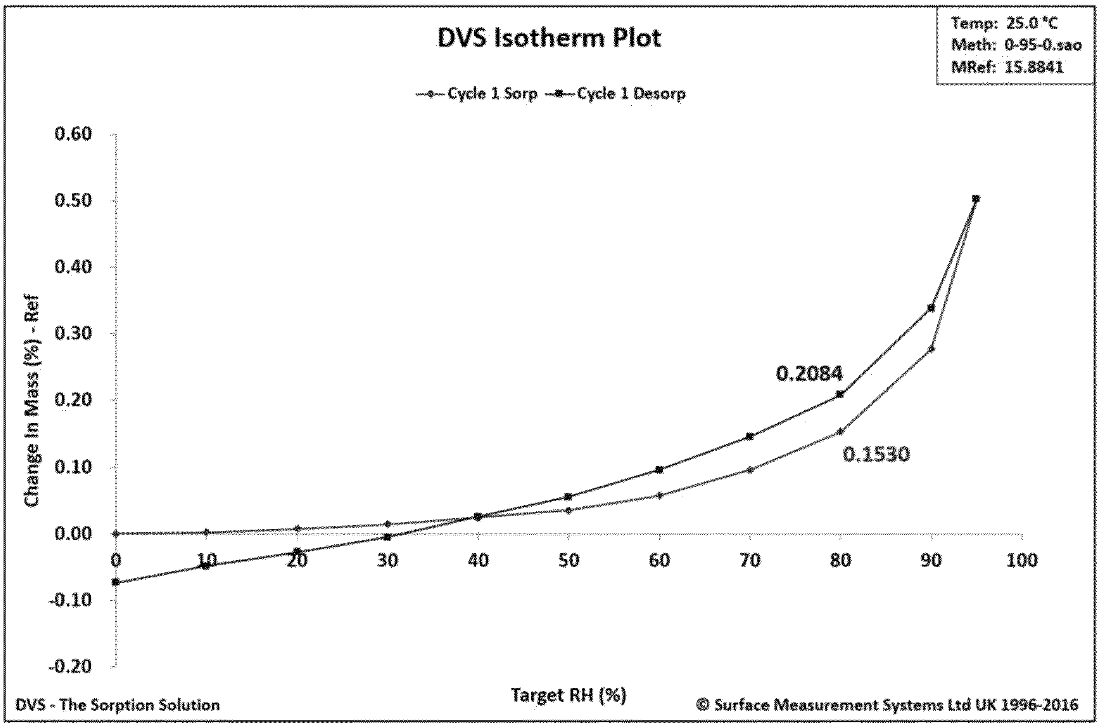

DVS Isotherm Plot
Temp: 25.0 °C
Meth: 0-95-0.sao
MRef: 15.8841
Cycle 1 Sorp
Cycle 1 Desorp
0.60
0.50
0.40
0.30
0.20
0.10
0.00
-0.10
-0.20
Change In Mass (%) - Ref
0.2084
0.1530
0
10
20
30
40
50
60
70
80
90
100
Target RH (%)
DVS - The Sorption Solution
© Surface Measurement Systems Ltd UK 1996-2016


FIG. 4

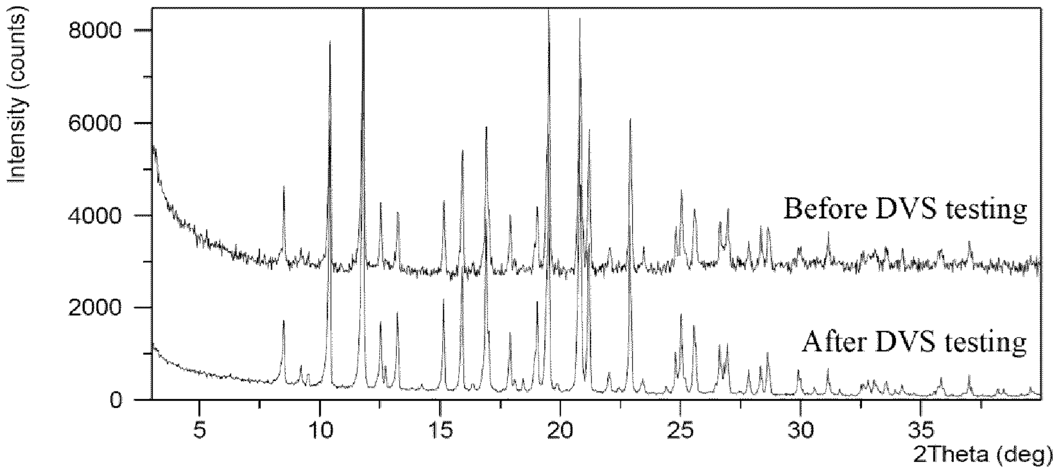
Intensity (counts)
8000
6000
4000
2000
0
5
10
15
20
25
30
35
2Theta (deg)
Before DVS testing
After DVS testing

FIG. 5

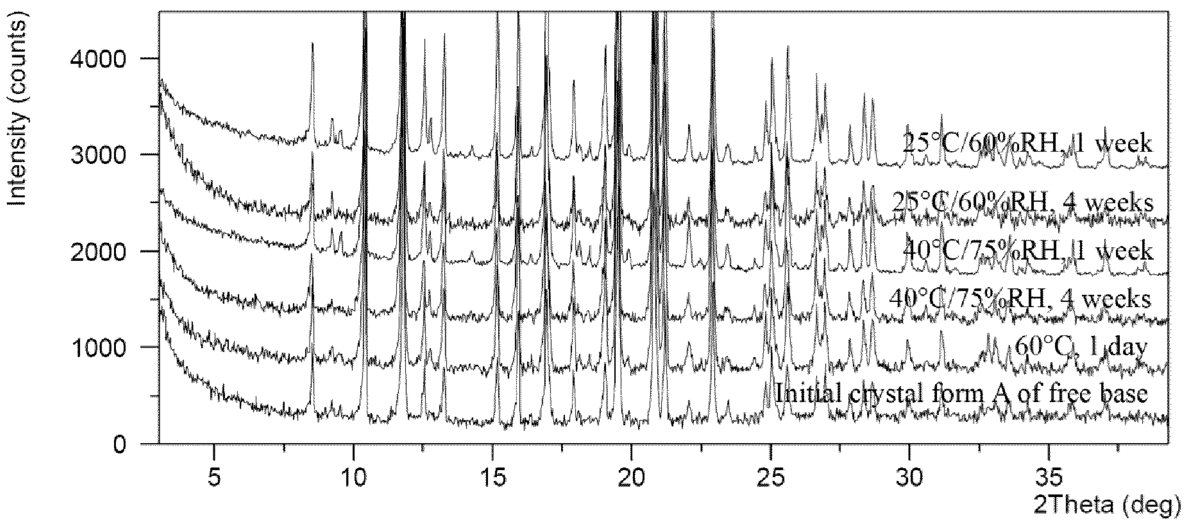
Intensity (counts)
4000
3000
2000
1000
0
5
10
15
20
25
30
35
2Theta (deg)
25°C/60%RH, 1 week
25°C/60%RH, 4 weeks
40°C/75%RH, 1 week
40°C/75%RH, 4 weeks
60°C, 1 day
Initial crystal form A of free base

FIG. 6

**INTERNATIONAL SEARCH REPORT**

International application No.
**PCT/CN2023/125214**

**A. CLASSIFICATION OF SUBJECT MATTER**

C07D 487/04(2006.01)i; A61K31/4985(2006.01)i; A61P25/00(2006.01)i; A61P29/00(2006.01)i

According to International Patent Classification (IPC) or to both national classification and IPC

**B. FIELDS SEARCHED**

Minimum documentation searched (classification system followed by classification symbols)

C07D, A61K, A61P

Documentation searched other than minimum documentation to the extent that such documents are included in the fields searched

Electronic data base consulted during the international search (name of data base and, where practicable, search terms used)

CNTXT, ENTXT, CNKI, STN(REGISTRY, MARPAT, CAPLUS): 咪唑, 吡嗪, 神经基肽, 噻二唑, 炎症, 精神病, 疼痛, 肥胖, 长春金赛, NK-3, imidazole, pyrazine, neurokinin, thiadiazole, inflammation, psychiatry, pain, obesity, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| PX | WO 2022222963 A1 (CHANGCHUN GENESCIENCE PHARMACEUTICAL CO., LTD.) 27 October 2022 (2022-10-27)<br>abstract, claims 1-16, and embodiments | 1-10 |
| A | CN 102906093 A (EUROSCREEN SA) 30 January 2013 (2013-01-30)<br>abstract, and claims 1 and 12 | 1-10 |
| A | CN 107405347 A (EUROSCREEN SA) 28 November 2017 (2017-11-28)<br>description, paragraphs 210-263 | 1-10 |
| A | WO 2022022680 A1 (SHANGHAI HANSOH BIOMEDICAL CO., LTD. et al.) 03 February 2022 (2022-02-03)<br>abstract, and claims 1 and 11 | 1-10 |
| A | WO 2014154895 A1 (EUROSCREEN SA) 02 October 2014 (2014-10-02)<br>abstract, and claims 1 and 12 | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 January 2024** | **01 February 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/125214**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **10**
because they relate to subject matter not required to be searched by this Authority, namely:

Claim 10 relates to a method for treatment of a disease. In the search report, a search is carried out on the basis of a corresponding pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/125214**

| Patent document cited in search report | Publication date (day/month/year) | Patent family member(s) | Publication date (day/month/year) |
|---|---|---|---|
| WO 2022222963 A1 | 27 October 2022 | CA 3214215 A1 | 27 October 2022 |
| | | EP 4293023 A1 | 20 December 2023 |
| | | KR 20230146639 A | 19 October 2023 |
| | | AU 2022263070 A1 | 26 October 2023 |
| CN 102906093 A | 30 January 2013 | US 2020125564 A1 | 23 April 2020 |
| | | US 2013023530 A1 | 24 January 2013 |
| | | US 8871761 B2 | 28 October 2014 |
| | | WO 2011121137 A1 | 06 October 2011 |
| | | HUE 032770 T2 | 30 October 2017 |
| | | US 2018194772 A1 | 12 July 2018 |
| | | US 10544150 B2 | 28 January 2020 |
| | | HRP 20170712 T1 | 28 July 2017 |
| | | LT 2552920 T | 12 June 2017 |
| | | MX 2012011132 A | 08 March 2013 |
| | | MX 342161 B | 19 September 2016 |
| | | US 2014371218 A1 | 18 December 2014 |
| | | US 9926325 B2 | 27 March 2018 |
| | | AU 2011234398 A1 | 13 September 2012 |
| | | AU 2011234398 A2 | 17 January 2013 |
| | | AU 2011234398 B2 | 23 October 2014 |
| | | AU 2011234398 C1 | 26 February 2015 |
| | | AU 2011234398 B8 | 05 March 2015 |
| | | EP 3176171 A1 | 07 June 2017 |
| | | CA 2793313 A1 | 06 October 2011 |
| | | CA 2793313 C | 23 January 2018 |
| | | KR 20130021384 A | 05 March 2013 |
| | | KR 101878888 B1 | 17 August 2018 |
| | | SI 2552920 T1 | 31 July 2017 |
| | | PL 2552920 T3 | 31 August 2017 |
| | | DK 2552920 T3 | 12 June 2017 |
| | | EA 201290940 A1 | 29 March 2013 |
| | | EA 023161 B1 | 29 April 2016 |
| | | RS 55997 B1 | 29 September 2017 |
| | | BR 112012025101 A2 | 20 June 2017 |
| | | BR 112012025101 B1 | 31 August 2021 |
| | | EP 2552920 A1 | 06 February 2013 |
| | | EP 2552920 B1 | 15 March 2017 |
| | | PT 2552920 T | 07 June 2017 |
| | | JP 2013523697 A | 17 June 2013 |
| | | JP 5822911 B2 | 25 November 2015 |
| | | ES 2627688 T3 | 31 July 2017 |
| CN 107405347 A | 28 November 2017 | WO 2016146712 A1 | 22 September 2016 |
| | | US 2023039022 A1 | 09 February 2023 |
| | | AU 2016232191 A1 | 31 August 2017 |
| | | AU 2016232191 B2 | 01 July 2021 |
| | | SG 11201707521 QA | 30 October 2017 |
| | | US 2020222399 A1 | 16 July 2020 |
| | | US 11478472 B2 | 25 October 2022 |
| | | US 2018289705 A1 | 11 October 2018 |
| | | US 10624890 B2 | 21 April 2020 |

Form PCT/ISA/210 (patent family annex) (July 2022)

**INTERNATIONAL SEARCH REPORT**
**Information on patent family members**

International application No.
**PCT/CN2023/125214**

| Patent document cited in search report | Publication date (day/month/year) | Patent family member(s) | Publication date (day/month/year) |
|---|---|---|---|
| | | JP 2018507874 A | 22 March 2018 |
| | | JP 6762312 B2 | 30 September 2020 |
| | | DK 3271015 T3 | 02 January 2024 |
| | | IL 254386 A0 | 30 November 2017 |
| | | IL 254386 B | 30 September 2020 |
| | | BR 112017019829 A2 | 29 May 2018 |
| | | EP 3271015 A1 | 24 January 2018 |
| | | EP 3271015 B1 | 27 September 2023 |
| | | EP 3271015 B8 | 01 November 2023 |
| | | CA 2977444 A1 | 22 September 2016 |
| | | KR 20170124566 A | 10 November 2017 |
| | | MX 2017011937 A | 01 February 2018 |
| | | EA 201791901 A1 | 30 April 2018 |
| | | EA 036709 B1 | 10 December 2020 |
| | | FI 3271015 T3 | 18 December 2023 |
| WO 2022022680 A1 | 03 February 2022 | AU 2021317809 A1 | 16 February 2023 |
| | | EP 4190784 A1 | 07 June 2023 |
| | | JP 2023537244 A | 31 August 2023 |
| | | KR 20230044443 A | 04 April 2023 |
| | | CA 3186086 A1 | 03 February 2022 |
| | | US 2023271969 A1 | 31 August 2023 |
| WO 2014154895 A1 | 02 October 2014 | ZA 201506234 B | 30 January 2019 |
| | | HK 1244273 A1 | 03 August 2018 |
| | | HUE 035870 T2 | 28 May 2018 |
| | | IL 241473 A0 | 30 November 2015 |
| | | IL 241473 B | 31 March 2019 |
| | | JP 2016515565 A | 30 May 2016 |
| | | JP 6316932 B2 | 25 April 2018 |
| | | KR 20210021406 A | 25 February 2021 |
| | | KR 102364835 B1 | 17 February 2022 |
| | | US 2016318941 A1 | 03 November 2016 |
| | | US 2017240551 A9 | 24 August 2017 |
| | | US 10030025 B2 | 24 July 2018 |
| | | EA 201591689 A1 | 29 January 2016 |
| | | EA 027570 B1 | 31 August 2017 |
| | | ES 2646488 T3 | 14 December 2017 |
| | | MX 2015013711 A | 26 February 2016 |
| | | MX 370000 B | 28 November 2019 |
| | | HK 1214816 A1 | 05 August 2016 |
| | | EP 3219715 A1 | 20 September 2017 |
| | | KR 20160007515 A | 20 January 2016 |
| | | KR 102218621 B1 | 22 February 2021 |
| | | JP 2018118988 A | 02 August 2018 |
| | | EP 2948455 A1 | 02 December 2015 |
| | | EP 2948455 B1 | 27 September 2017 |
| | | NZ 712802 A | 28 August 2020 |
| | | US 2015232471 A1 | 20 August 2015 |
| | | US 9422299 B2 | 23 August 2016 |
| | | CY 1119576 T1 | 07 March 2018 |
| | | RS 56501 B1 | 28 February 2018 |

Form PCT/ISA/210 (patent family annex) (July 2022)

**INTERNATIONAL SEARCH REPORT**
**Information on patent family members**

International application No.
**PCT/CN2023/125214**

| Patent document cited in search report | Publication date (day/month/year) | Patent family member(s) | | | Publication date (day/month/year) |
|---|---|---|---|---|---|
| | | SG | 11201508005 | XA | 29 October 2015 |
| | | NO | 2948455 | T3 | 24 February 2018 |
| | | US | 2017095472 | A1 | 06 April 2017 |
| | | US | 9987274 | B2 | 05 June 2018 |
| | | CA | 2907809 | A1 | 02 October 2014 |
| | | CA | 2907809 | C | 04 May 2021 |
| | | PL | 2948455 | T3 | 31 January 2018 |
| | | DK | 2948455 | T3 | 20 November 2017 |
| | | DK | 2948455 | T5 | 19 March 2018 |
| | | BR | 112015024907 | A2 | 18 July 2017 |
| | | BR | 112015024907 | B1 | 09 May 2023 |
| | | PT | 2948455 | T | 15 November 2017 |
| | | AU | 2014242906 | A1 | 22 October 2015 |
| | | AU | 2014242906 | A2 | 05 November 2015 |
| | | AU | 2014242906 | B2 | 26 July 2018 |
| | | SI | 2948455 | T1 | 29 December 2017 |
| | | LT | 2948455 | T | 27 November 2017 |
| | | HRP | 20171688 | T1 | 29 December 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202211282020 A
- WO 202311315851 A
- CN 2022087947